Europäisches Patentamt

European Patent Office

Office européen des brevets

(11) Veröffentlichungsnummer: **0 418 553 A1**

# (12) EUROPÄISCHE PATENTANMELDUNG

(21) Anmeldenummer: 90115703.2

(22) Anmeldetag: 16.08.90

(51) Int. Cl.⁵: **A61K 31/64**, A61K 9/18, A61K 9/00

(30) Priorität: 23.08.89 DE 3927882

(43) Veröffentlichungstag der Anmeldung:
27.03.91 Patentblatt 91/13

(84) Benannte Vertragsstaaten:
**AT BE CH DE DK ES FR GB GR IT LI LU NL SE**

(71) Anmelder: **Bauer, Kurt H. Prof. Dr.**
**Im Finkeler 4**
**W-7800 Freiburg-Tiengen(DE)**

Anmelder: **Keller, Manfred, Dr.**
**Giessenstrasse 10**
**W-7800 Freiburg(DE)**

(72) Erfinder: **Bauer, Kurt H. Prof. Dr.**
**Im Finkeler 4**
**W-7800 Freiburg-Tiengen(DE)**
Erfinder: **Keller, Manfred, Dr.**
**Giessenstrasse 10**
**W-7800 Freiburg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

(54) **Hochwirksame, schnell resorbierbare Zubereitungsformen von Glibenclamid, Verfahren zu ihrer Herstellung und ihre Verwendung.**

(57) Die Erfindung betrifft hochwirksame, den Wirkstoff schnell freisetzende flüssige und feste pharmazeutische Zübereitungsformen zur Behandlung der Zuckerkrankheit, insbesondere:

1. flüssige Zübereitungsformen von Glibenclamid bestehend aus 1 Teil Glibenclamid und 4 - 1500 Teilen Glykol, wie z.B. Propylenglykol, Hexylenglykol, Di, -Tri- oder Polyethylenglykol mit einem Molekulargewicht von 76 bis 600, gegebenenfalls 0,5 bis 3 Mol einer alkalisch reagierenden Substanz und sonstigen Arzneimittelzusatzstoffen.

2. feste Zübereitungsformen von Glibenclamid bestehend aus 1 Teil Glibenclamid, 4-35 Teilen Polyethylenglykol mit einem Molekulargewicht von 600 bis zu 1950, 0,5 bis 3 Mol Ammoniak, gegebenenfalls verdünnt in Alkohol, 1 - 30 Teilen pharmakologisch unbedenklicher Hilfsstoffe, Trägermaterialien oder sonstigen Arzneimittelzusatzstoffen.

Die Erfindung betrifft ferner ein Verfahren zur Herstellung einer molekulardispersen Lösung bzw. Wirkstofflegierung von Glibenclamid oder einer anderen schwerlöslichen Substanz aus der Reihe der Sulfonylharnstoffe oder von Substanzen bzw. Verbindungen mit ähnlichen chemisch-physikalischen Eigenschaften.

EP 0 418 553 A1

## HOCHWIRKSAME, SCHNELL RESORBIERBARE ZUBEREITUNGSFORMEN VON GLIBENCLAMID. VERFAHREN ZU IHRER HERSTELLUNG UND IHRE VERWENDUNG

*Einleitung*

Glibenclamid (INN) ist ein Sulfonylharnstoffderivat mit der chemischen IUPAC-Bezeichnung: N-4-2-(5-Chlor-2-Methoxybenzamido)-äthyl - phenylsulfonyl-N´-cyclohexylharnstoff. Glibenclamid ist eine geruchlose, kristalline, weiße Substanz, die in Wasser und Ether praktisch unlöslich, in Alkohol und Chloroform nur mäßig löslich ist. Mit Alkalien bildet sie Salze, die jedoch im Vergleich zu Tolbutamid, einer ebenfalls blutzuckersenkenden Substanz eine sehr begrenzte Löslichkeit in Wasser besitzen. Das Molekulargewicht beträgt 494.02, der Schmelzpunkt liegt bei 172 bis 174° C (vergl. R. Gröning: Arzneistoffprofile und Bioverfügbarkeitsdaten von Fertigarzneimitteln, Arzneistoffmonographie Glibenclamid, Deutscher Apothekerverlag, Stuttgart, Seite 1 bis 7, 1987; Euglucon[R] N: Semi-Euglucon[R] N: Pharmakologische und klinische Daten, Präparateinformation Böhringer Mannheim GmbH und Hoechst AG, Frankfurt/Main, 1984).

Glibenclamid besitzt blutzuckersenkende Eigenschaften und wird heute auf breiter Basis weltweit zur Behandlung der Zuckerkrankheit (Diabetes mellitus) eingesetzt. Bei der unter Glibenclamid eintretenden Blutzuckersenkung lassen sich zwei Mechanismen unterscheiden, nämlich ein pankreatischer und extrapankreatischer Effekt (vergl. die oben erwähnte Firmenschrift der Fa. Böhringer Mannheim und Hoechst AG).

Die pankreatische Wirkung führt zu einer Verstärkung der Insulinsekretion durch eine erhöhte Ansprechbarkeit der Beta-Zellen des Pankreas auf Glucose. Der extrapankreatische Effekt zeigt sich bei einer Insulinresistenz in einer Verstärkung der Insulinwirkung durch:
- Erhöhung der Insulinempfindlichkeit und Insulinbindung des Zielgewebes:
- direkte Wirkung auf die Insulinrezeptoren im Sinne einer Vermehrung.

Aufgrund der guten Wirksamkeit und Verträglichkeit von Glibenclamid besitzen diese Präparate heute die größte Bedeutung bei der oralen Behandlung der Zuckerkrankheit.

*Stand der Technik und Anforderungen für eine optimale Wirkstoffzubereitung*

Es ist bekannt und in Untersuchungen belegt, daß zur Erzielung eines therapeutischen Effekts nicht nur die Wirksubstanz, sondern auch deren pharmazeutischtechnologische Formulierung, auch als Galenik bezeichnet, von großer Bedeutung ist (vergl. H. Blume et al.: Zur Bioverfügbarkeit und pharmakodynamischen Aktivität handelsüblicher Glibenclamid-Fertigarzneimittel,

1. Mitteilung: Bioequivalenzprüfung an gesunden Probanden unter oraler Kohlenhydratbelastung: Pharm. Ztg., 130 , 1062-1069, 1985;
2. Mitteilung: Untersuchungen der glibenclamidinduzierten Veränderungen der Insulinkonzentration im Serum und der Blutglucosewerte an gesunden Probanden. Pharm. Ztg., 130 , 1070-1078, 1985;
3. Mitteilung: Bioequivalenzprüfung an gesunden Probanden unter Dauerinfusion von Glucoselösung. Pharm. Ztg., 130 , 2606-2610, 1985).

Aus diesen Untersuchungen geht hervor, daß Glibenclamid aufgrund seiner geringen Wasserlöslichkeit und Lösungsgeschwindigkeit zu den biopharmazeutisch problematischen Arzneisubstanzen zählt. Die Wasserlöslichkeit ist zudem pH-abhängig, im sauren Bereich ist Glibenclamid praktisch wasserunlöslich.

Bekannt ist ebenfalls, daß die Lösungsgeschwindigkeit von der Teilchengröße bzw. von der Ausdehnung der Partikeloberfläche abhängt (vergl. H. Borchert et al.: Zur pharmazeutischen Qualität von Glibenclamid in Abhängigkeit von der Teilchengröße, Pharmazie, 31 , 307-309, 1976). Bereits vor Jahren wurden deshalb Anstrengungen unternommen, die schlechte Löslichkeit und Lösungsgeschwindigkeit zu verbessern. Gefunden wurde, daß Zübereitungen mit mikronisierterm, d.h. feinstzerkleinertem Glibenclamid (mittlere Partikelgröße $\geq$ 5um) vorallem in Gegenwart von Tensiden eine verbesserte Wirkstofffreisetzung und Bioverfügbarkeit zeigten (R. Gröning ibid., H. Borchert et al., ibid.).

In der DE-OS 23 48 334 wird eine schnell resorbierbare Zübereitungsform von Glibenclamid und ein Verfahren zu ihrer Herstellung beschrieben. Glibenclamid wurde durch Feinstvermahlung auf eine große, möglichst definierte Oberfläche oder durch feinstes Ausfällen aus organischen Lösungsmitteln in Dispergiermittel zu gut und schnell resorbierbaren Zubereitungen verarbeitet. Mit den in der DE-OS 23 48 334 aufgeführten Methoden wurden Feinstkristallisate mit einer Oberflächengröße von 3 bis 10 m² erhalten. Untersuchungen haben gezeigt, daß durch eine Oberflächenvergrößerung die Löslichkeit und Lösungsgeschwindigkeit erhöht werden, wodurch auch die Bioverfügbarkeit verbessert wurde.

Mit einer Glibenclamid-Tablette (Semi-Euglucon[R] N) mit 1,75 mg Wirkstoffgehalt konnten diesselben Glibenclamid-Wirkstoffspiegel erzielt werden wie mit einer Tablette, die 2,5 mg Glibenclamid enthielt (Euglucon$^R$ 2,5). Hierbei bestanden keine Unterschiede bezüglich der maximalen Serumkonzentrationen ($C_{max}$ für beide Formulierungen ca. 100 ng/ml), während $T_{max}$ (ca. 1 Stunde) etwa 1,5 Stunden früher erreicht wurde als mit der alten Formulierung Semi-Euglucon$^R$ 2,5. Aus diesen Untersuchungen und aus vergleichenden Studien zur Austauschbarkeit von glibenclamidhaltigen Fertigarzneimitteln ist also ersichtlich, daß die Löslichkeit von Glibenclamid, bestimmt durch die Korn- bzw. Oberflächengröße, für die Bioverfügbarkeit und Wirksamkeit der verschiedenen Präparate von großer Bedeutung ist und bestimmte Präparate zur Substitution nicht geeignet sind (vergl. H. Blume et al.: Untersuchungen zur therapeutischen Relevanz und zur Chargenhomogenität glibenclamidhaltiger Fertigarzneimittel, Pharm. Ztg., 132 , 2352-2362, 1987).

Es ist desweiteren bekannt, daß Temperaturbelastung bei der Herstellung von Arzneiformen, die Wirkstoffstabilität zu beeinflussen vermag. In der DE-OS 23 55 743 sind Beispiele aufgeführt, die zeigen, daß die Verarbeitung von Glibenclamid bei erhöhten Temperaturen, z.B durch Einarbeitung in Schmelzen von höhermolekularen Polyethylenglykolen der Stabilität abträglich sind.

Darüberhinaus wurde in jüngerer Zeit diskutiert, inwieweit aus Polyethylenglykolen, insbesondere unter Temperaturbelastung, möglicherweise kanzerogenverdächtiges Ethylenoxid freigesetzt wird. Nach einem Erfahrungssatz aus der chemisch-physikalischen Chemie ist bekannt, daß eine Temperatererhöhung um 10°C etwa zu einer Verdoppelung der Reaktionsgeschwindigkeit führt. Die Höhe der Temperatur während des Herstellungsvorganges sollte also so gering als möglich gehalten werden. In diesem Zusammenhang ist auch verständlich, weshalb z.B. das Bundesgesundheitsamt vom pharmazeutischen Hersteller den Nachweis fordert, daß Polyethylenglykole für pharmazeutische Fertigprodukte nicht mehr als 1 ppm Ethylenoxid enthalten.

Nur wenige Handelspräparate, wie z.B. Euglucon$^R$ N Tabletten besitzen in-vitro eine relativ schnelle Wirkstoffabgabe von Glibenclamid und demzufolge in-vivo eine hinreichend rasche und gute Resorption bzw. Bioverfügbarkeit. Eine rasche Resorption des Wirkstoffs aus der Arzneiform bietet folgende Vorteile:

1. Aus Gründen der Einnahmementalität der Patienten kann die Arzneiform gleichzeitig mit der Nahrung verabreicht werden.

2. Der Wirkstoff steht, genau abgestimmt, bedarfs- und zeitgerecht in vivo zur Verfügung, womit das Auftreten von erhöhten oder stark schwankenden Blutzuckerwerten verhindert werden kann.

3. Hyperglykämien, bedingt durch Nahrungsaufnahme oder sonstige Einflüsse, können verhindert bzw. rasch normalisiert werden.

Arzneiformen, die diesen Ansprüchen nach einer raschen Wirkstofffreisetzung nicht gerecht werden, bergen die Gefahr von Hyperglykämien, wenn keine ausreichende Menge Wirkstoff, aufgrund unzureichender Löslichkeit bzw. Freisetzung, für die Resorption zur Verfügung steht. Voraussetzung für eine rasche Resorption ist jedoch, daß der Wirkstoff aus der Arzneiform möglichst in gelöster bzw. kolloidal gelöster oder molekulardispers verteilter Form Für die Freisetzung zur Verfügung steht. Nur wenn diese Bedingungen erfüllt sind, kann der Wirkstoff rasch resorbiert und damit wirksam werden.

## *Zielsetzung für die Erfindung*

Der vorliegenden Erfindung lag die Aufgabe zugrunde, hochwirksame, schnell resorbierbare Zubereitungsformen von schwerlöslichen, blutzuckersenkenden Sulfonylharnstoffderivaten, wie z.B. Glibenclamid zu entwickeln und Verfahren zu ihrer Herstellung zur Verfügung zu stellen.

Der schwerlösliche Wirkstoff Glibenclamid sollte mit einem einfachen Verfahren und pharmakologisch, toxikologisch unbedenklichen Hilfsstoffen möglichst molekulardispers in Lösung gebracht werden. Hierdurch wird der Wirkstoff feinst verteilt und besitzt infolge der großen Oberfläche eine gute Löslichkeit. Der molekulardispes verteilte Wirkstoff kann zur Obrfächenvergrößerung auf übliche pharmazeutische Trägermaterialien aufgezogen oder auch darin eingebettet werden, so daß eine Wirkstoff-Hilfsstoff-Legierung erhalten wird. Durch das molekulardisperse in Lösung bringen, können schwerlösliche Sulfonylharnstoffderivate, wie z.B. Glibenclamid schnell aus dem Magen-Darm-Trakt resorbiert werden. Zur Vermeidung von Hyperglykämien steht Glibenglamid somit im Bedarfsfalle oder im Zusammenhang mit der Nahrungsaufnahme (z.B. durch Kohlenhydratzufuhr) sofort zur Verfügung.

Das Verfahren zur Herstellung der erfindungsgemäßen Zubereitungsformen sollte technisch einfach durchzuführen sein, keine aufwendigen Apparaturen benötigen und keine komplizierten Verfahrensschritte aufweisen. Darüberhinaus soll es eine schonende Behandlung des Wirkstoffs z. B. bei niederen Temperaturen gewährleisten und seine Stabilität nicht negativ beeinflussen. Die Herstellung sollte mit handelsüblichen, pharmakopökonformen Hilfsstoffen und einfachen Mitteln möglich sein. Darüberhinaus sollten durch das Herstellungsverfahren Wirk- bzw. Hilfsstoffe in ihren chemisch- physikalischen Eigenschaften nicht negativ beeinflußt werden.

## Gegenstand der Erfindung

Im Gegensatz zu den in der DE-OS 23 55 743 aufgeführten wasserlöslichen Trägerstoffen, für die ein Schmelzpunkt von über 50°C gefordert wird, wurde überraschenderweise gefunden, daß niedermolekulare Polyethylenglykole Glibenclamid besser zu lösen vermögen. Von Vorteil erweist sich desweiteren, daß dadurch die externe Wärmezufuhr auf Temperaturen unter 50°C gehalten werden kann. Mit niedermolekularen Polyethylenglykolen können deshalb mögliche Stabilitätsbeeinträchtigungen von Glibenclamid reduziert bzw. vermieden werden.

Überraschend wurde nun festgestellt, daß die benötigte Menge Polyethylenglykol unter Bezugnahme der Beispiele 1 und 2 in der DE-OS 23 55 743 um das bis zu 10-fache erniedrigt werden kann, wenn der Glibenclamid-Polyethylenglykolmischung 0,5 bis 3, vorzugsweise 1 Mol Ammoniak, konzentriert (35%ig) oder verdünnt in Ethanol (10%ig), zugesetzt wird.

Das erfindungsgemäße Herstellungsverfahren ermöglicht also nicht nur eine Reduktion der erforderlichen Hilfsstoffmenge, sondern stellt auch gleichzeitig ein wirkstoff- und hilfsstoffschonendes, einfaches Verfahren zur Lösung von schwerlöslichen Sulfonylharnstoffdrivaten, wie z.B. Glibenclamids dar.

Die Erfindung betrifft ein Verfahren zur Herstellung von flüssigen, hochwirksamen, schnell resorbierbaren Zubereitungsformen, das dadurch gekennzeichnet ist, daß 1 Teil Glibenclamid in 3 bis 1500 Teilen Propylenglykol, Hexylenglykol, Di-, Tri- oder Polyethylenglykole mit einem Molekulargewicht von 76,1 bis 600 oder Mischungen davon, unter Zugabe von 0,5 bis zu 3 Mol, vorzugsweise etwa 1 Mol alkalisch reagierender Substanz, wie z.B. Ammoniak, die gegebenenfalls in in 0,5 bis 20 Teilen Ethanol gelöst wird, unter Rühren in Lösung gebracht wird.

Mit diesem Verfahren ist es überraschenderweise möglich, flüssige Darreichungsform zu herzustellen, die als Tropflösungen eingesetzt werden können. Die flüssigen Zubereitungen eignen sich auch zur Abfüllung in Weichgelatine-Kapseln.

Die Erfindung betrifft flüssige , den Wirkstoff schnell freisetzende Zubereitungen von Glibenclamid, die dadurch gekennzeichnet sind, daß sie aus einer Lösung, bestehend aus 1 Teil schwerlöslichem Sulfonylharnstoffdrivat, wie z.B. Glibenclamid, 3 bis 1500 Teilen Glykolen, vorzugsweise Propylenglykol, Hexylenglykol, Di,- Tri,-oder Polyethylenglykol mit einem Molekulargewicht von 76,1 bis zu 600 oder Gemischen davon und gegebenenfalls 0,5 bis 3 Mol, vorzugsweise etwa 1 Mol einer alkalisch reagierenden Substanz, wie Alkali- bzw. Erdalkalihydroxide oder Ammoniak, bezogen auf das Glibenclamid, sowie weiteren geeigneten, Hilfs- oder auch Aromastoffen besteht.

Beide pharmazeutischen Darreichungsformen, Tropfen und Weichgelatine-Kapseln, sind bislang für die Therapie als Handelsprodukte nicht erhältlich. Die Tropflösung erlaubt eine individuelle, dem Krankheitszustand und der Nahrungszufuhr angepaßte Dosierung. Aufgrund der Tatsache, daß in der erfindungsgemäßen Zubereitung der Wirkstoff molekulardispers gelöst vorliegt, steht er für die Resorption direkt zur Verfügung und erlaubt dadurch eine rasche Resorption und optimale Bioverfügbarkeit.

Die mit dem erfindungsgemäßen Verfahren auch herstellbaren festen Mischungen enthalten Glibenclamid in molekulardisperser Form d.h. als feste Lösung bzw. Legierung.

Die Erfindung betrifft ebenfalls feste , den Wirkstoff schnell freisetzende Zubereitungsformen von schwerlöslichen, blutzuckersenkenden Sulfonylharnstoffderivaten, wie z.B. Glibenclamid, die dadurch gekennzeichnet sind, daß sie aus einer festen Lösung bzw. Legierung aus 1 Teil Glibenclamid, 4 bis 35 Teilen Polyethylenglykol mit einem Molekulargewicht von 200 bis zu 1950 bzw. Gemischen davon und 0,5 bis 3 Mol, vorzugsweise etwa 1 Mol Ammoniak, bezogen auf das Glibenclamid und gegebenenfalls 1 bis 50 Teilen pharmakologisch unbedenklicher Hilfsstoffe oder Trägermaterialien sowie üblichen Arzneizusatzstoffen bestehen.

Darreichungsformen, die Glibenclamid in den erfindungsgemäßen Zubereitungsformen enthalten, bieten einen sehr großen Vorteil, weil sie zusammen mit der Nahrung oder unmittelbar nach der Nahrungsaufnahme, zum Beispiel mit oder nach dem Frühstück, verabfolgt werden können. Aufgrund der raschen Auflösung von Glibenclamid in molekulardisperser oder kolloidaler Form, kann der Wirkstoff rasch resorbiert werden, d.h. er steht bedarfs- und zeitgerecht z.B. gleichzeitig mit der Kohlenhydrataufnahme zur Verfügung.

Für die praktische Anwendung bedeutet dies, daß der Patient keine komplizierten und Compliance gefährdenden Einnahmevorschriften zu beachten hat, da der Wirkstoff im Organismus dann zur Verfügung steht, wenn er gebraucht wird.

## Herstellungsverfahren

Das Verfahren zur Herstellung **flüßiger** Dareichungsformen ist denkbar einfach. Das schwerlösliche Sulfonylharnstoffdrivat, wie z.B. Glibenclamid

4

wird in einem Rührkessel in 4 bis 1500 Teilen flüssigem Propylen- Hexylen- oder Polyehtylen-glykol mit einem Molekulargewicht von 76,1 bis 600 unter Rühren suspendiert und solange mit alkalisch reagierender Substanz, wie z.B. Ammoniak, ethanolischer Natronlauge) etc. versetzt, bis eine klare, homogene Lösung erhalten wird. Diese Lösung kann dann mit weiteren geeigneten Hilfsstoffen, wie z.B. Wasser, Süß- Aroma- und Kon servierungsstoffen gemischt werden und dann als flüssige pharmazeutischen Darreichungsform (z.B. als Tropflösung) therapeutisch zur Blutzuckersenkung verwendet werden.

Zur Herstellung **fester** Darreichungsformen wird der Wirkstoff unter gelinder Wärmezufuhr (kleiner 50°C) in 4-35 Teilen Polyethylenglykol mit einem Molekulargewicht von 400-1950 unter Rühren suspendiert und solange mit Ammoniak, konzentriert oder in Ethanol verdünnt, versetzt, bis eine klare Lösung resultiert. Erfindungsgemäß kann man auch Mischungen aus Propylen- und Hexylenglykol sowie Polyethylenglykolen mit unterschiedlichen Molekulargewichten verwenden. Man kann beispielsweise Glibenclamid in 1 bis 6 Teilen Polyethylenglykol 200 suspendieren, zu der Suspension Ammoniak zugeben und dann mit 5 bis 20 Teilen Polyethylenglykol 1500 unter gelinden Erwärmen (35 - 50°C) vermischen. Die warme Lösung kann im Wirbelschichtgranulator auf geeignete Hilfsstoffe aufgezogen oder durch rasches Abkühlen bzw. Abschrecken verfestigt werden. Dies kann dadurch bewerkstelligt werden, daß man die warme Lösung auf Kühlbleche, Kühlwalzen oder Kühltrommeln ausgießt oder Verfahren der Sprüherstarrung in Sprüh- oder Kühltürmen oder sonstige geeignete Verfahren verwendet.

Ammoniak und überschüssiger Alkohol können, falls vorhanden, anschließend im Vakuum (Primärtrocknung), gegebenenfalls unter leichter Wärmezufuhr (Sekundärtrocknung), entfernt werden.

Die erkaltete, feste Lösung kann anschließend gegebenenfalls zerbrochen oder zerkleinert werden und mit üblichen Hilfsstoffen zu Granulaten oder anderen festen Darreichungsformen, wie z.B. Tabletten verarbeitet werden. Bevorzugt ist es, die Lösung auf Füllmittel, wie z.B. Mannit, Lactose oder Trägersubstanzen, wie z.B. mikrokristalline Cellulose oder Sprengmittel oder Mischungen davon aufzuziehen. Mit einem solchen Vorgehen wird der Wirkstoff quasi molekulardispers bzw. als Wirkstofflegierung auf einer großen Oberfläche verteilt.

Das erfindungsgemäß molekulardispers verteilte, als feste Lösung aufgezogene Glibenclamid, ist so fein dispergiert und so gut benetzbar, daß eine weitere Steigerung der Lösungsgeschwindigkeit theoretisch und praktisch nicht mehr möglich ist. Nach dem Noyes-Whitney-Gesetz ist die Lösungsgeschwindigkeit umso schneller, je größer die benetzbare Oberfläche der zu lösenden Partikel ist.

Eine stärkere Zerkleinerung als eine molekulardisperse ist praktisch nicht mehr möglich. Die erfindungsgemäße Zubereitung besitzt deshalb als feste Lösung bzw. Wirkstofflegierung einen nicht mehr zu übertreffenden Dispersitätsgrad. Die Verwendung einer festen Lösung oder Wirkstofflegierung in festen pharmazeutischen Darreichungsformen ermöglicht deshalb eine ideale Freisetzung, rasche Resorption und optimale Bioverfügbarkeit.

*Beispiele für die erfindungsgemäßen Zubereitungen*

1. 10 g Glibenclamid werden in 500 ml Diglykol unter Rühren suspendiert und solange mit 4% iger ethanolischer Natronlauge versetzt, bis eine klare Lösung entsteht.

2. 1,5 g Glibenclamid werden unter Rühren in 1500 ml Triglykol gelöst.

3. 1 g Glibenclamid wird in 4 ml Polyethylenglykol 300 unter Rühren suspendiert und solange mit 10% iger ethanolischer Ammoniaklösung versetzt, bis eine klare Lösung resultiert. Eventuell überschüssiger Alkohol und Ammoniak können im Vakuum, gegebenenfalls unter gelinder Wärmezufuhr, entfernt werden.

4. 1 g Glibenclamid werden in 40 g Propylenglykol vermischt und solange mit 4% iger ethanolischer Kalilauge versetzt, bis eine klare Lösung erhalten wird.

5. 2 g Glibenclamid werden in 1000 ml Polyethylenglykol 400 gelöst.

6. 10 g Glibenclamid werden in 70 g Polyethylenglykol 1000 unter Erwärmen gelöst und mit 50 ml Ethanol, das 10% Ammoniak enthält, vermischt. Die warme Lösung wird auf 100 g Sprengmittel/mikrokristalline Cellulose (1:10) aufgesprüht und auf 8°C abgekühlt. Im Vakuum werden anschließend, gegebenenfalls unter Erwärmen, überschüssiger Ammoniak und Alkohol entfernt.

7. 10 g Glibenclamid werden mit 50 g Polyethylenglykol 1500 unter Rühren und gelindem Erwärmen vermischt und solange mit konzentrierter Ammoniaklösung versetzt, bis eine klare Lösung erhalten wird. Diese wird in diesem Zustand verfestigt, indem sie auf ein vorgekühltes Blech ausgegossen und auf -10°C abgekühlt wird. Die erstarrte Lösung wird anschließend in grobe Partikel zerbrochen und gegebenenfalls im Vakuum von überschüßigem Ammoniak befreit.

8. 10 g Glibenclamid werden mit 5 g Polyethylenglykol 600 verrührt und unter gelindem Erwärmen mit 200 g Polyethylenglykol 1500 vermischt. Es wird solange isopropanolhaltiger Ammoniak (10% G/V) zugesetzt, bis eine klare Lösung erhalten wird. Diese wird dann auf eine vorgewärmte Mischung aus 50 g mikrokristalliner Cellulose, 50 g Lactose und 25 g Sprengmittel aufgezogen.

9. 10 g Glibenclamid werden unter gelindem Erwärmen in 350 g Polethylenglykol 1000 vermischt und solange tropfenweise mit Ammoniak versetzt, bis eine klare Lösung erhalten wird. Diese wird mit 300 g einer vorgekühlten Mischung aus gleichen Teilen Lactose, mikrokristalline Cellulose und Maisstärke vermischt und auf 15° C abgekühlt.

**Ansprüche**

1. Hochwirksame, den Wirkstoff schnell freisetzende Zubereitung von Glibenclamid, dadurch gekennzeichnet, daß sie aus einer Lösung, bestehend aus 1 Teil Glibenclamid, 4-1500 Teilen Glykolen, vorzugsweise Propylenglykol, Hexylenglykol, Di-, Tri- oder Polyethylenglykol mit einem Molekulargewicht von 76,1 bis zu 600 oder Gemischen davon und gegebenenfalls 0,5 bis 3 Mol, vorzugsweise etwa 1 Mol einer alkalisch reagierenden Substanz, wie Alkali- bzw. Erdalkalihydroxide oder Ammoniak, bezogen auf das Glibenclamid, sowie gegebenenfalls weiteren geeigneten, Hilfs - oder Aromastoffen besteht.

2. Zubereitungsform nach Anspruch 1, dadurch gekennzeichnet, daß als Hilfsstoffe Ethanol, Ethanol-Wasser-Mischungen, Wasser, Isotonisierungszusätze, Puffersubstanzen und als Aromastoffe Zuckeraustauschstoffe, wie z.B. Fructose, Sorbit, Mannit, Xylit, Saccharin, Aspartam, etc. sowie gegebenenfalls Aromastoffe und Konservierungsmittel, wie z.B. Parabene eingesetzt werden.

3. Zubereitungsform nach Anspruch 1, dadurch gekennzeichnet, daß sie in Form von üblichen flüssigen pharmazeutischen Darreichungsformen, wie z.B. Lösungen (Tropfen, Säften, Injektions- oder Infusionslösungen) alleine oder auch in Kombination mit anderen blutzuckersenkenden Medikamenten, wie z.B. anderen Sulfonylharnstoffderivaten oder dem Pseudotetrasaccharid Acarbose vorliegt.

4. Verfahren zur Herstellung von flüssigen hochwirksamen, schnell resorbierbaren Zubereitungsformen nach Anspruch 1, 2 und 3, dadurch gekennzeichnet, daß 1 Teil Glibenclamid in 4 bis 1500 Teilen Propylenglykol, Hexylenglykol, Di-, Tri- oder Polyethylenglykolen mit einem Molekulargewicht von 76,1 bis 600 oder Mischungen davon, gegebenenfalls unter Zugabe von 0,5 bis zu 3 Mol, vorzugsweise etwa 1 Mol alkalisch reagierender Substanz, die gegebenenfalls in in 0,5 bis 20 Teilen Ethanol gelöst wird, unter Rühren in Lösung gebracht wird, und gegebenenfalls weitere geeignete Hilfs- oder Aromastoffe zugegeben werden.

5. Hochwirksame, den Wirkstoff schnell freisetzende Zubereitungsform von Glibenclamid, dadurch gekennzeichnet, daß sie aus einer festen Lösung bzw. Legierung aus 1 Teil Glibenclamid, 4-35 Teilen Polyethylenglykol mit einem Molekulargewicht von 200 bis zu 1950 bzw. Gemischen davon und 0,5 bis 3 Mol, vorzugsweise etwa 1 Mol Ammoniak, bezogen auf das Glibenclamid und gegebenenfalls 1 bis 30 Teilen pharmakologisch unbedenklicher Hilfsstoffe oder Trägermaterialien sowie üblichen Arzneizusatzstoffen besteht.

6. Zubereitungsform nach Anspruch 5 dadurch gekennzeichnet, daß sie als Hilfsstoffe Alkohole, wie z.B. Ethanol, Glycerol und als Trägermaterialien, z.B. mikrokristalline Cellulose, Stärke, Lactose, Mannit, Celluloseetherderivate sowie Sprengmittel, z.B. Acdisol, Natriumhydrogencarbonat oder auch Mischungen davon enthält.

7. Zubereitungsform nach Anspruch 5 und 6, dadurch gekennzeichnet, daß sie in Form von üblichen pharmazeutischen Darreichungsformen mit sofortiger oder modifizierter Wirkstoffabgabe, wie z.B. Granulaten, Pellets, Weich- oder Hartgelatinekapseln, Dragees, Tabletten mit und ohne Überzug alleine oder auch in Kombination mit anderen blutzuckersenkenden Medikamenten, wie z.B. anderen Sulfonylharnstoffderivaten oder dem Pseudotetrasaccharid Acarbose vorliegt.

8. Verfahren zur Herstellung einer hochwirksamen, schnell resorbierbaren Zubereitung von blutzuckersenkenden Wirkstoffen, wie z.B. Glibenclamid oder anderen Sulfonylharnstoffderivaten mit ähnlichen chemisch-physikalischen Eigenschaften, nach Anspruch 5, 6 und 7 dadurch gekennzeichnet, daß 1 Teil Wirkstoff in 4 bis 35 Teilen Polyethylenglykol mit einem Molekulargewicht von 200 bis 1950 unter Zugabe von 0,5 bis 3 Mol, vorzugsweise etwa 1 Mol Ammoniak, das gegebenfalls in 0,5 bis 10 Teilen Ethanol verdünnt wird, unter Rühren bei Temperaturen unter 50° C gelöst und mit den in Anspruch 6 genannten Hilfsstoffen (Mannit, Lactose, mikrokristalline Cellulose, Stärke, Sprengmittel, Formentrennmittel, etc) so vermischt wird, daß eine feste Lösung bzw. Wirkstoff-Hilfsstoff-Legierung erhalten wird.

9. Verfahren nach Ansprüchen 4 und 8, dadurch gekennzeichnet, daß als alkalisch reagierende Substanz Ammoniak und als Lösungs- bzw. Verdünnungsmittel Alkohole, vorzugsweise Ethanol, Isopropanol oder Glycerol, Propylenglykol Hexylenglykol verwendet werden.

10. Verfahren zur Herstellung von hochwirksamen, schnell resorbierbaren Zubereitungsformen nach mindestens einem der Ansprüche 4, 8 oder 9,, dadurch gekennzeichnet, daß neben Glibenclamid auch andere schwerlösliche Sulfonylharnstoffderivate damit kolloidal oder molekulardispers gelöst werden können.

11. Verwendung der hochwirksamen, schnell resorbierbaren Zübereitungsformen von Glibenclamid oder anderen Sulfonylharnstoffderivaten nach mindestens einem der Ansprüche 1 bis 3, 5 bis 7 oder der nach den Verfahren der Ansprüche 4, 8 oder 9 hergestellten Zübereitungsformen zur Herstellung von Arzneimitteln zur Behandlung der Zuckerkrankheit (Diabetes mellitus).

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 128 482   (Dr. KARL THOMAE) <br> * Ansprüche 1,2,6; Seite 7, Zeilen 4-9; Beispiele 23,24,32 * <br> — — — | 8,9,11 | A 61 K 31/64 <br> A 61 K 9/18 <br> A 61 K 9/00 |
| X | EP-A-0 086 468   (HOECHST) <br> * Ansprüche 1-10; Seite 4, Zeilen 5,12-17; Seite 5, Zeilen 10,22-25 * <br> — — — | 1,2,4-9, 11 | |
| X | DE-A-3 228 384   (Dr. RENTSCHLER ARZNEIMITTEL) <br> * Ansprüche 1,2,5,6,7; Seite 5, Absatz 1; Seite 4, letzter Absatz; Seite 7, Absatz 3 * <br> — — — — — | 1-5 | |

| RECHERCHIERTE SACHGEBIETE (Int. Cl.5) |
|---|
| A 61 K |

Der vorliegende Recherchenbericht wurde für alle Patentansprüche erstellt

| Recherchenort | Abschlußdatum der Recherche | Prüfer |
|---|---|---|
| Den Haag | 11 Dezember 90 | PEETERS J.C. |